# EUROPÄISCHE PATENTANMELDUNG

(11) **EP 4 342 429 A2**
(43) Veröffentlichungstag der Anmeldung: **27.03.2024**
(21) Anmeldenummer: 24156520.9
(22) Anmeldetag: 10.02.2022
(51) Int. Cl.: A61F 2/24

(54) **SYSTEM ZUR BEHANDLUNG, PRÄVENTION UND/ODER ZUM ERSATZ EINER HERZKLAPPE, INSBESONDERE EINER ENTZÜNDETEN, INFIZIERTEN, THROMBOSIERTEN ODER DEGENERIERTEN HERZKLAPPE**

(30) Priorität: 10.02.2021 DE 102021000811
(62) Teilanmeldung aus: 22706034.0
(71) Anmelder: Devie Medical GmbH, 07743 Jena (DE)
(72) Erfinder: Figulla, Hans-Reiner, 07749 Jena (DE); Seidel, Raphael Andreas, 99428 Weimar (DE); Chavalla, Sharath Chandra, 07747 Jena (DE)
(74) Vertreter: Meissner Bolte Partnerschaft mbB

(57) **Zusammenfassung**

Kathetergeführte wirkstofffreisetzende Herzklappenprothesen, die durch besondere Konstruktionsmerkmale wie beispielsweise herunterklappbare Kompartimentierungsclips, diffusionsdichte Strukturen und wirkstofffreisetzende Matrices ausgestattet sind, um infektiöse Herzklappenerkrankungen lokal zu behandeln. Dabei ersetzt die erfindungsgemäße Herzklappenprothese einerseits die Herzklappenfunktion der infizierten Klappe und therapiert andererseits gleichzeitig die Infektion über eine lokale Wirkstofffreisetzung.

## Beschreibung

Gegenwärtig sind zahlreiche Stent-Systeme zum Stützen und Offenhalten von Gefäßen, sowie zur Einbringung bzw. zum Ersatz von Gefäßklappen, wie beispielsweise Herzklappen, vorhanden. Diese Systeme können einerseits chirurgisch, beispielsweise durch eine Operation am offenen Herzen oder minimalinvasiv, und andererseits katheterbasiert direkt über einen arteriellen oder venösen Zugang ins Herzkreislaufsystem eingebracht werden. Die kathetergeführten Implantate besitzen häufig ein ballonexpandierbares Metallgerüst oder einen aus einer Formgedächtnislegierung bestehenden, selbstexpandierbaren Metallrahmen für die Stents bzw. Herzklappen. Weiterhin sind z.T. dünne Schichten oder gewebeartige Strukturen an- oder aufgebracht, die einerseits das Einwachsen des Implantats erleichtern sollen und andererseits das Lumen des Implantats offenhalten und insbesondere bei Herzklappen einen Blutfluss um die Klappe herum (paravalvuläres Leck) verhindern sollen. Die über einen Katheter eingebrachten Herzklappenimplantate zeichnen sich dadurch aus, dass die Belastung für einen Patienten gegenüber dem operativen, den Thorax eröffnenden Eingriff, reduziert ist. Bei Infektionen an einer nativen oder prothetischen Herzklappe, der sogenannten nativen oder prothetischen Endokarditis (NVE, PVE), die mit einer Destruktion der Klappen einhergehen ist ein kathetergeführter Herzklappenersatz absolut kontraindiziert, da dadurch infizierte Bereiche von der Blutzirkulation abgetrennt werden und davon ausgegangen werden kann, dass sich die Infektion ausweitet und sich die Situation für den Patienten verschlechtert. Darüber hinaus kann es beim Einsetzen von herkömmlichen kathetergeführten Herzklappen dazu kommen, dass infektiöse Bakterienauflagerungen an den nativen Herzklappensegeln (sogenannte Vegetationen) in den Blutstrom abreißen und embolisieren.

Der offene chirurgische Eingriff unter Einsatz einer Herz-Lungen-Maschine, der trotz Multimorbidität bei der Mehrzahl der Patienten mit Endokarditis durchgeführt werden muss, kann das infizierte Material, im Gegensatz zum kathetergeführten Herzklappenersatz, entfernen. Allerdings sind diese Operationen bei der infektiösen Endokarditis aufgrund der mit der Endokarditis verbundenen Komorbidität der Patienten mit hohen Eingriffsrisiken verbunden, so dass bei der NVE und PVE eine mittlere Krankenhaussterblichkeit von ungefähr 20% auftritt. Der aktuelle Stand der Technik ist weiterhin in der veröffentlichten Patentanmeldung WO 2020/074130 A1 wiedergegeben. Die in WO 2020/074130 A1 vorgestellten Rahmen für Herzklappenprothesen zum Einsatz bei entzündeten, thrombosierten oder degenerierten Herzklappen besitzen einen zweiteiligen Aufbau, wobei zwischen dem einen, die neuen Herzklappensegel tragenden Teil und dem anderen, als Widerlager fungierenden Teil die infizierten Bereiche kompartimentiert und behandelt werden. Diese Zweiteilige Lösung weist folgende Nachteile auf: Die Einbringung von zwei Teilen ist zeitaufwändiger und technisch kompliziert, da für eine effiziente Kompartimentierung während der kathetergeführten Implantation eine form- und/oder kraftschlüssige Verbindung zwischen beiden Teilen erreicht werden muss. Auch kleinere Lücken zwischen beiden Teilen könnten ein zumindest teilweises Auswaschen der Wirkstoffe im kompartimentierten Vorlumen begünstigen und so eine Behandlung mit ausreichend hohen Konzentrationen gefährden. Weiterhin könnten zweiteilige Lösungen an den Verbindungs- oder Berührungsstellen beider Teile durch die ständige pulsatile Bewegung des schlagenden Herzens Abrieberscheinungen zeigen, die zu Materialermüdung oder Embolisationen führen könnten.

### Grundsätzliche technische Lösung des Problems

Die Erfindung löst das o.g. klinische Problem, indem kathetergeführte Herzklappenprothesen so konstruiert sind, dass die infizierten Bereiche mittels Kompartimentierungsclips vom Blutstrom abgetrennt werden und auf der Herzklappenprothese wirkstofffreisetzende Matrices angebracht sind, die die Entzündung durch Kompartimentierung lokal therapieren (Wirkstoff-freisetzende Herzklappenprothese). Darüber hinaus ist die Herzklappenprothese so konstruiert, dass infektiöse Auflagerungen (Vegetationen) mit den Kompartimentierungsclips und daran befestigten Strukturen umgriffen und somit eine Embolisation verhindert wird. Ausführungsformen dieser kathetergeführten Herzklappenkonstruktionen erlauben auch einer insgesamt seltenen (1 - 1,5% im ersten Jahr postoperativ) auftretende postoperative PVE vorzubeugen.

Die erfindungsgemäßen Lösungen sind in den Nummern angegeben.

Nach einem ersten Aspekt betrifft die Erfindung eine Verankerung für Herzklappenprothesen, insbesondere für Herzklappenprothesen zur Behandlung, Prävention, und/oder zum Ersatz einer Herzklappe, insbesondere einer entzündeten, infizierten, thrombosierten und/oder degenerierten Herzklappe, wobei die Verankerung eine Grundform aufweist und zum minimal-invasiven Einführen der Verankerung in den Körper des Patienten reversibel und insbesondere durch elastische Umformung und/oder Kompression zumindest von Bereichen oder Komponenten der Verankerung in eine Zuführungsform überführbar ist, wobei die Verankerung folgendes aufweist: ein Stentgerüst, welches dazu ausgebildet ist, mindestens zwei Herzklappensegel an einer Innenseite zu befestigen, wobei das Stentgerüst ein proximales Ende und ein distales Ende aufweist; und mindestens zwei Kompartimentierungsclips, welche jeweils an einem ersten Endbereich mit dem Stentgerüst verbunden sind und einen freien, zweiten Endbereich aufweisen, wobei in der Grundform der Verankerung die Kompartimentierungsclips eine Bogenform zwischen den ersten und zweiten Endbereichen aufweisen.

Nach einer weiteren Ausführungsform überlappen, in der Grundform der Verankerung, die Kompartimentierungsclips das Stentgerüst zumindest teilweise überlappen, insbesondere in radialer Richtung des Stentgerüsts.

Nach einer weiteren Ausführungsform wobei die Kompartimentierungsclips derart elastisch umklappbar sind, dass sich in der Zuführungsform der Verankerung die Kompartimentierungsclips zumindest im Wesentlichen flach zwischen den ersten und zweiten Endbereichen erstrecken.

Nach einer weiteren Ausführungsform weisen die Kompartimentierungsclips Arme auf, welche in der Grundform der Verankerung zumindest bereichsweise verdrillt sind.

Nach einer weiteren Ausführungsform weisen die Kompartimentierungsclips eine erste Clipoberfläche und eine der ersten Clipoberfläche gegenüberliegende zweite Clipoberfläche auf, wobei sowohl in der Grundform als auch in der Zuführungsform der Verankerung die erste Clipoberfläche am freien, zweiten Endbereich der Kompartimentierungsclips zumindest im Wesentlichen radial nach innen, insbesondere in Richtung einer Längsachse der Verankerung, gerichtet ist.

Nach einer weiteren Ausführungsform ist sowohl in der Grundform als auch in der Zuführungsform der Verankerung die zweite Oberfläche am zweiten Endbereich der Kompartimentierungsclips zumindest im Wesentlichen radial nach außen gerichtet.

Nach einer weiteren Ausführungsform ist die Verankerung dazu ausgebildet ist, in einem implantierten Zustand der Verankerung native Segel bzw. Leaflets und/oder infektiöse und/oder thrombotische Auflagerungen/Vegetationen der erkrankten Herzklappe zwischen dem Stentgerüst und den Kompartimentierungsclips zu halten, insbesondere einzuklemmen.

Nach einer weiteren Ausführungsform weist die Verankerung eine Trennstruktur auf, die derart mit dem Stentgerüst und den Kompartimentierungsclips verbunden ist, dass im implantierten Zustand der Verankerung die Trennstruktur die zu behandelnden Bereiche der nativen Segel bzw. Leaflets vom Blutstrom trennt.

Nach einer weiteren Ausführungsform weist die Trennstruktur eine erste Strukturoberfläche, welche in der Zuführungsform der Verankerung radial nach innen, insbesondere in Richtung einer Längsachse der Verankerung, gerichtet ist, und eine der ersten Strukturoberfläche gegenüberliegende, zweite Strukturoberfläche auf, und wobei zumindest die zweite Strukturoberfläche eine Matrix aufweist, welche antibiotische, antithrombotische und/oder thrombolytische Wirkstoffe enthält und abgeben kann.

Nach einer weiteren Ausführungsform sind das Stentgerüst und die Kompartimentierungsclips aus einem Stück gefertigt.

Nach einer weiteren Ausführungsform sind das Stentgerüst und/oder die Kompartimentierungsclips aus einem komprimierbaren Werkstoff gefertigt.

Nach einer weiteren Ausführungsform sind das Stentgerüst und/oder die Kompartimentierungsclips aus einem selbstexpandierenden Werkstoff gefertigt.

Nach einer weiteren Ausführungsform sind das Stentgerüst und die Kompartimentierungsclips aus mindestens zwei miteinander verbundenen oder verbindbaren Stücken eines selbstexpandierbaren Werkstoffs gefertigt.

Nach einer weiteren Ausführungsform sind das Stentgerüst und die Kompartimentierungsclips aus mindestens zwei miteinander verbundenen oder verbindbaren Stücken gefertigt, wobei das Stentgerüst aus einem ballonexpandierbaren Werkstoff und die Kompartimentierungsclips aus einem selbstexpandierbaren Werkstoff gefertigt sind.

Nach einer weiteren Ausführungsform bestehen das Stentgerüst und/oder die Kompartimentierungsclips aus einer Formgedächtnislegierung, insbesondere Nitinol.

Nach einer weiteren Ausführungsform überlappen in der Grundform der Verankerung die mindestens zwei Kompartimentierungsclips das Stentgerüst derart, dass kein Teil der Kompartimentierungsclips über das distale Ende des Stentgerüsts hinausragt.

Nach einem weiteren Aspekt betrifft die Erfindung einen Katheter zum Einführen einer Verankerung für Herzklappenprothesen in den Körper eines Patienten, wobei mit Hilfe des Katheters die Verankerung in einer Zuführungsform minimal-invasiv in den Körper des Patienten einführbar und im Implantationsort an der erkrankten Herzklappe in einen implantierten Zustand überführbar ist, und wobei die Verankerung folgendes aufweist:
- ein Stentgerüst welches dazu ausgebildet ist, mindestens zwei Herzklappensegel oder Leaflets an einer Innenseite zu befestigen, wobei das Stentgerüst ein proximales Ende und ein distales Ende aufweist;
- mindestens zwei Kompartimentierungsclips, welche mit dem Stentgerüst verbunden sind, und welche in einer Grundform der Verankerung das Stentgerüst zumindest teilweise überlappen, um native Segel bzw. Leaflets und/oder infektiöse und/oder thrombotische Auflagerungen/Vegetationen der erkrankten Herzklappe zwischen dem Stentgerüst und den Kompartimentierungsclips zu halten, insbesondere einzuklemmen,
wobei der Katheter eine Katheterspitze aufweist, welche über eine Handhabe des Katheters derart manipulierbar ist, dass das Implantat schrittweise von der Katheterspitze freisetzbar ist.

Nach einer weiteren Ausführungsform ist die Katheterspitze derart geteilt ausgebildet, dass die mindestens zwei Kompartimentierungsclips schrittweise, nacheinander von der Katheterspitze freisetzbar sind, und dann folgend das Stentgerüst freigesetzt werden kann.

Nach einer weiteren Ausführungsform weist die geteilte Katheterspitze eine angeschrägte Hülse auf, welche dazu ausgebildet ist, die Verankerung während des Einbringens zu überdecken, und welche mit Hilfe der Handhabe zurückziehbar ist, um die Kompartimentierungsclips schrittweise freizusetzen.

Nach einer weiteren Ausführungsform ist der Katheter dazu ausgebildet, die Verankerung über die Handhabe zu rotieren, um Kompartimentierungsclips auf die Taschen/Segel/Leaflets der nativen Klappe auszurichten.

Nach einem weiteren Aspekt betrifft die Erfindung ein System zur Behandlung, Prävention und/oder zum Ersatz einer Herzklappe, insbesondere einer entzündeten, infizierten, thrombosierten oder degenerierten Herzklappe, wobei das System eine der oben stehenden Verankerung und einen der oben stehenden Katheter nach aufweist, wobei die Verankerung dazu ausgebildet ist, insbesondere in der Zuführungsform der Verankerung in der Katheterspitze aufnehmbar zu sein, und wobei die Katheterspitze ausgebildet ist, die Verankerung, insbesondere in der Zuführungsform der Verankerung, aufzunehmen.

Nach einem weiteren Aspekt betrifft die Erfindung ein Verfahren zur Behandlung, Prävention, und/oder zum Ersatz einer Herzklappe, insbesondere einer entzündeten, infizierten, thrombosierten oder degenerierten Herzklappe, wobei das Verfahren folgendes aufweist:
- Einführen einer Verankerung mit Hilfe eines Katheters;
- Freisetzen der Kompartimentierungsclips;
- Ausrichten der Kompartimentierungsclips auf einer ersten Seite der nativen Segel/Leaflets der erkrankten Herzklappe;
- Freisetzen des Stentgerüsts auf einer zweiten Seite der nativen Segel, welche der ersten Seite gegenüberliegt.

Nach einer weiteren Ausführungsform weist das Verfahren ferner den Verfahrensschritt der Freigabe von antibiotischen, antithrombotischen, thrombolytischen und/oder zellwachstumshemmenden Wirkstoffen auf, wobei die Freigabe in-situ erfolgt.

Nach einer weiteren Ausführungsform werden die Kompartimentierungsclips schrittweise, nacheinander freigesetzt werden.

Nach einem weiteren Aspekt betrifft die Erfindung eine Verankerung für Herzklappenprothesen, insbesondere für Herzklappenprothesen zur Behandlung, Prävention, und/oder zum Ersatz einer Herzklappe, insbesondere einer entzündeten, infizierten, thrombosierten oder degenerierten Herzklappe, wobei die Verankerung eine Grundform aufweist, und wobei die Verankerung zum minimal-invasiven Einführen der Verankerung in den Körper des Patienten reversibel und insbesondere durch elastische Umformung und/oder Kompression zumindest von Bereichen oder Komponenten der Verankerung in eine Zuführungsform überführbar ist, wobei die Verankerung folgendes aufweist:
- ein Stentgerüst, welches dazu ausgebildet ist, mindestens zwei Herzklappensegel an einer Innenseite zu befestigen, wobei das Stentgerüst ein proximales Ende und ein distales Ende aufweist;
- mindestens zwei Stabilisierungsbögen, welche über das distale Ende des Stentgerüsts hinausragen und dazu ausgebildet sind, sich im implantierten Zustand der Verankerung derart an einer Gefäßwand abzustützen, dass die Verankerung nach Freisetzung der Stabilisierungsbögen zentral in dem zu implantierenden Blutgefäß ausgerichtet ist.

Nach einer weiteren Ausführungsform sind die mindestens zwei Stabilisierungsbögen in ihrer Länge und/oder Form so ausgebildet, dass sie im Röntgenbild unterscheidbar sind und bei der Implantation nacheinander freigesetzt werden können.

Nach einer weiteren Ausführungsform weisen die Stabilisierungsbögen ein proximales Ende, das mit dem distalen Ende des Stentgerüsts verbunden ist, und ein distales Ende, das über distale Ende des Stentgerüsts hinausragt, auf, wobei die Stabilisierungsbögen am distalen Ende durch Streben, Rauten oder andere Elemente miteinander verbunden sind, welche dazu ausgebildet sind, die radiale Stabilität zu vergrößern und eine Zentralisierung der Verankerung im zu implantierbaren Blutgefäß zu ermöglichen.

Nach einer weiteren Ausführungsform weist die Verankerung eine Trennstruktur auf, die derart mit dem Stentgerüst verbunden ist, dass die Trennstruktur im expandierten Zustand der Verankerung die zu behandelnden Bereiche der nativen Segel bzw. Leaflets vom Blutstrom trennt.

Nach einer weiteren Ausführungsform weist die Trennstruktur zumindest außenseitig eine Matrix auf, welche antibiotische, antithrombotische und/oder thrombolytische Wirkstoffe enthält und abgeben kann.

Nach einer weiteren Ausführungsform sind das Stentgerüst und die Stabilisierungsbögen aus einem Stück gefertigt.

Nach einer weiteren Ausführungsform sind das Stentgerüst und/oder die Stabilisierungsbögen aus einem komprimierbaren Werkstoff gefertigt.

Nach einer weiteren Ausführungsform sind das Stentgerüst und/oder die Stabilisierungsbögen aus einem selbstexpandierenden Werkstoff gefertigt sind.

Nach einer weiteren Ausführungsform bestehen das Stentgerüst und/oder die Stabilisierungsbögen aus einer Formgedächtnislegierung, insbesondere Nitinol.

Nach einer weiteren Ausführungsform weist die Verankerung mindestens zwei Kompartimentierungsclips, welche jeweils an einem ersten Endbereich mit dem Stentgerüst verbunden sind und einen freien, zweiten Endbereich aufweisen, wobei die Kompartimentierungsclips in der Grundform der Verankerung eine Bogenform zwischen den ersten und zweiten Endbereichen aufweisen.

Nach einer weiteren Ausführungsform überlappen die Kompartimentierungsclips in der Grundform der Verankerung das Stentgerüst zumindest teilweise.

Nach einer weiteren Ausführungsform sind die Kompartimentierungsclips derart umklappbar, dass sich die Kompartimentierungsclips in der Zuführungsform der Verankerung zumindest im Wesentlichen flach zwischen den ersten und zweiten Endbereichen erstrecken.

Nach einem weiteren Aspekt betrifft die Erfindung eine Verankerung für Herzklappenprothesen, insbesondere für Herzklappenprothesen zur Behandlung, Prävention, und/oder zum Ersatz einer Herzklappe, insbesondere einer entzündeten, infizierten, thrombosierten oder degenerierten Herzklappe, wobei die Verankerung eine Grundform aufweist, und wobei zum minimal-invasiven Einführen der Verankerung in den Körper des Patienten die Verankerung reversibel und insbesondere durch elastische Umformung und/oder Kompression zumindest von Bereichen oder Komponenten der Verankerung in eine Zuführungsform überführbar ist, wobei die Verankerung folgendes aufweist:
- ein Stentgerüst welches dazu ausgebildet ist, mindestens zwei Herzklappensegel bzw. Leaflets an einer Innenseite zu befestigen, wobei das Stentgerüst ein proximales Ende und ein distales Ende aufweist;
- mindestens zwei Kompartimentierungsclips, welche mit dem Stentgerüst verbunden sind und, in der Grundform der Verankerung, das Stentgerüst zumindest teilweise überlappen, um native Segel und/oder infektiöse und/oder thrombotische Auflagerungen/Vegetationen der erkrankten Herzklappe, zwischen dem Stentgerüst und den Kompartimentierungsclips zu halten, insbesondere einzuklemmen;
- eine Trennstruktur, die derart mit dem Stentgerüst und den Kompartimentierungsclips verbunden ist, dass die Trennstruktur, im implantierten Zustand, die zu behandelnden Bereiche der nativen Segel vom Blutstrom trennt.

Nach einer weiteren Ausführungsform weist die Trennstruktur eine erste Strukturoberfläche, welche in der Zuführungsform radial nach innen, insbesondere in Richtung einer Längsachse der Verankerung, gerichtet ist, und eine der ersten Strukturoberfläche gegenüberliegende, zweite Strukturoberfläche auf, wobei zumindest die zweite Strukturoberfläche eine Matrix aufweist, welche antibiotische, antithrombotische und/oder thrombolytische Wirkstoffe enthält und abgeben kann.

Nach einer weiteren Ausführungsform ist die mindestens eine wirkstoffhaltige Matrix bioresorbierbar ist.

Nach einer weiteren Ausführungsform sind das Stentgerüst und die Kompartimentierungsclips aus einem Stück gefertigt.

Nach einer weiteren Ausführungsform sind das Stentgerüst und/oder die Kompartimentierungsclips aus einem komprimierbaren Werkstoff gefertigt.

Nach einer weiteren Ausführungsform sind das Stentgerüst und/oder die Kompartimentierungsclips aus einem selbstexpandierbaren Werkstoff gefertigt.

Nach einer weiteren Ausführungsform sind das Stentgerüst und die Kompartimentierungsclips aus mindestens zwei miteinander verbundenen oder verbindbaren Stücken eines selbstexpandierbaren Werkstoffs gefertigt.

Nach einer weiteren Ausführungsform sind das Stentgerüst und die Kompartimentierungsclips aus mindestens zwei miteinander verbundenen verbindbaren Stücken gefertigt sind, und das Stentgerüst aus einem ballonexpandierbaren Werkstoff und die Kompartimentierungsclips aus einem selbstexpandierbaren Werkstoff gefertigt.

Nach einer weiteren Ausführungsform bestehen das Stentgerüst und/oder die Kompartimentierungsclips aus einer Formgedächtnislegierung, insbesondere Nitinol.

Nach einer weiteren Ausführungsform weisen die mindestens zwei Kompartimentierungsclips an einen ersten Endbereich, der mit dem Stentgerüst verbunden ist, und einen freien, zweiten Endbereich auf, wobei die Kompartimentierungsclips in der Grundform der Verankerung eine Bogenform zwischen den ersten und zweiten Endbereichen aufweisen.

Nach einer weiteren Ausführungsform überlappen die Kompartimentierungsclips, in der Grundform der Verankerung, das Stentgerüst zumindest teilweise.

Nach einer weiteren Ausführungsform sind die Kompartimentierungsclips derart umklappbar, dass sich die Kompartimentierungsclips in der Zuführungsform der Verankerung zumindest im Wesentlichen flach zwischen den ersten und zweiten Endbereichen erstrecken.

Nach einer weiteren Ausführungsform weist die Verankerung drei Kompartimentierungsclips auf.

Nach einer weiteren Ausführungsform sind die mindestens zwei Kompartimentierungsclips im expandierten Zustand der Verankerung radial voneinander beabstandet und über das Stentgerüst miteinander verbunden.

Nach einer weiteren Ausführungsform weist das Stentgerüst an seinem proximalen Ende eine erste Zellstruktur aus Streben und an seinem distalen Ende eine zweite Zellstruktur aus Streben auf, wobei die erste und die zweite Zellstruktur unterschiedlich ausgebildet sind.

Nach einer weiteren Ausführungsform weist die erste Zellstruktur eine höhere Dichte an Streben auf als die zweite Zellstruktur.

Nach einer weiteren Ausführungsform ist die Verankerung aus einem Rohr aus Formgedächtnislegierung geschnitten.

Nach einem weiteren Aspekt betrifft die Erfindung eine Herzklappenprothese, insbesondere für Herzklappenprothesen zur Behandlung, Prävention, und/oder zum Ersatz einer Herzklappe, insbesondere einer entzündeten, infizierten, thrombosierten oder degenerierten Herzklappe, wobei die Herzklappenprothese folgendes aufweist:
- eine oben beschriebene Verankerung;
- mindestens zwei Herzklappensegel, welche an der Innenseite des Stentgerüsts der Verankerung befestigt sind.

Die mit der Erfindung vorgestellten technischen Umsetzungen beinhaltet folgende Neuheiten und bietet folgende Vorteile gegenüber herkömmlichen kathetergeführten Herzklappenprothesen:
Der kathetergeführte Herzklappenersatz wird auch bei Patienten möglich, die eine bakterielle Endokarditis haben. Dieser weitgehend atraumatische Eingriff kann mit geringeren Risiken bei Patienten mit hoher Komorbidität, durchgeführt werden. Die durch die lokale Applikation erreichbaren antibakteriellen Wirkstoffkonzentrationen im Gewebe, sind deutlich höher, als die durch systemische Gaben erzielt werden kann. Systemische Antibiotikagaben sind begrenzt durch toxische Arzneimittelnebenwirkungen.

Ausführungsformen der erfindungsgemäßen Konstruktion einer solchen kathetergeführten Herzklappenprothese können das Risiko einer postoperativen Endokarditis (siehe oben) auf Grund einer prophylaktischen Langzeitantibiotikaabgabe reduzieren, indem die Besiedlung der Herzklappenprothese mit Mikroorganismen, insbesondere mit Bakterien, während der Phase des Einheilens, vor einer Infektion geschützt ist.

Die erfindungsgemäßen Konstruktionen der Herzklappenprothese erlaubt auch die Behandlung einer Herzklappenundichtigkeit (z.B. Aortenklappeninsuffizienz) als auch einer Herzklappenverengung (Stenose), indem die Fixation des Herzklappenimplantats über Kompartimentierungsclips an den nativen Herzklappensegeln erfolgt.

Die Erfindung erweitert somit das gegenwärtige Indikationsspektrum der kathetergeführten Herzklappenoperation entscheidend, indem die Therapie und Prophylaxe einer infektiösen Endokarditis (sowohl einer NVE als auch einer PVE) möglich wird.

Eine wesentliche Neuerung für zur Therapie und/oder Prophylaxe der Endokarditis bestimmten Herzklappenprothesen im Vergleich zu Dokument WO 2020/074130 A1 ist, dass die hier vorgestellte erfindungsgemäße Herzklappenprothese aus nur einem Teil aufgebaut ist. Dadurch werden folgende Vorteile erreicht: Die Herzklappenprothesen können in einem Schritt und somit mit weniger Zeit- und Materialaufwand implantiert werden, weil nicht zwei Teile form - oder kraftschlüssig gegeneinander ausgerichtet werden müssen. Weiterhin ergibt sich nicht das Problem, dass in der gesamten Zirkumferenz an den Verbindungs- bzw. Kontaktstellen mit den beschriebenen Problemen entstehen. Die erfindungsgemäßen einteiligen Herzklappenprothesen schaffen eine lückenlose Verbindung der beiden Bereiche, die innerhalb (luminal) und außerhalb (abluminal) der infizierten und ggf. mit infektiösen Auflagerungen (Vegetationen) belegten nativen Herzklappensegel umfassen und kompartimentieren. Die Kompartimentierung wird dadurch verbessert und es können bei den hier vorgestellten einteiligen Herzklappenprothesen höhere lokale Wirkstoffkonzentrationen erreicht werden.

Verschiedene Ausführungsformen der erfindungsgemäßen Herzklappe enthalten stets ein einziges zu implantierendes Metallgerüst, wobei dieses eine Gerüst (z.B. bei der Herstellung) auch aus mehreren Teilen zusammengesetzt sein kann, um beispielsweise verschiedene Materialien, um bestimmte Materialeigenschaften (selbstexpandierbar, ballonexpandierbar) zu kombinieren und/oder um bestimmte Stabilitäten oder Formgebungen umzusetzen. Für die Verbindung der einzelnen Teile zu dem einen Metallgerüst können verschiedenste Verfahren, u.a. Schweißen, Kleben, Nähen, Stecken etc. zum Einsatz kommen. Neben der Möglichkeit, dieses eine Metallgerüst auch aus verschiedenen Teilen zusammenzusetzen, sind ausdrücklich auch Ausführungsformen erwähnt, die aus einem einzigen Material und einem einzigen Stück gefertigt sind (beispielsweise ein aus einem einzigen Rohr einer Formgedächtnislegierung laser-geschnittenes Werkstück, dass in einem nachfolgenden Formgebungsprozess in Form gebracht wird).

Bei der kathethergeführten Implantation von Herzklappenprothesen - insbesondere, wenn wie z.B. bei einem transfemoralen Einführen des Katheters ins Blutsystem die Herzklappenprothese einen längeren Weg durch die Blutgefäße bis zum Implantationsort vorgeschoben werden muss - ist es auf Grund der begrenzten anatomischen Verhältnisse vorteilhaft, wenn die Herzklappenprothesen zur Implantation auf einen möglichst kleinen Durchmesser komprimiert ("gecrimpt") werden können. Würden die Strukturen und Materialien von zweiteiligen Herzklappenprothesen (Stentgerüst und Widerlagerstruktur) für den Ersatz von infizierten Herzklappen lediglich in einem Teil kombiniert werden, so würde die resultierende einteilige Lösung konsequenterweise auch im komprimierten/gecrimpten Zustand eine deutliche Umfangszunahme erfahren. Die hier vorgestellten erfindungsgemäßen einteiligen Herzklappenprothesen sind jedoch so aufgebaut, dass die Herzklappenprothese speziell konstruierte Kompartimentierungsclips enthält, welche bei Aufnahme in den Katheter also vor der Implantation, in longitudinaler (axialer) Richtung nach oben gebogen und zusammen mit dem Stentgerüst komprimiert werden können und so das Material nicht auf einer axialen Höhe übereinanderliegt, sondern über die axiale Länge verteilt wird. Dies ermöglicht die Einbringung des Einteilers mit gängigen Katheterdurchmessern. Während der Implantation werden die nach oben gebogenen Kompartimentierungsclips nach unten entlassen, wobei diese Freisetzung schrittweise erfolgen kann. Eine schrittweise Freisetzung kann erforderlich sein, da das Umklappen der (ggf. z.Z. noch komprimierten) bogenartigen Kompartimentierungsclips mit einer vorübergehenden Umfangszunahme einhergeht.

Neben dem Umklappen der Kompartimentierungsbögen können die Bögen in anderen erfindungsgemäßen Ausführungsformen auch schrittweise nach unten geschoben werden, sodass keine starke Umfangszunahme während dieses Schrittes des nach-unten-Ausrichtens erfolgt.

Bei der Implantation können mit den nach unten ausgerichteten Kompartimentierungsbögen, die mit einer durchgehenden Trennschicht und ggf. mit einer wirkstoffhaltigen Matrix verbunden sind, beim nach-unten-Schieben der teilweise noch komprimierten Herzklappenprothese die an den nativen Herzklappensegeln anhaftenden/aufgelagerten infektiösen Auflagerungen (Vegetationen) zusammen mit den infizierten Herzklappensegeln selbst umgreifen und einfangen. Dabei werden die Bögen der Kompartimentierungsclips bis in die Taschen der Herzklappen, die von den nativen Herzklappensegeln mit der Gefäßwand gebildet werden, eingeführt. Beim nachfolgenden Expandieren und Freisetzen des Stentgerüsts wird der umgriffene Bereich weiter komprimiert und die umgriffenen Bereiche werden so kompartimentiert. Die mindestens eine wirkstoffhaltige Matrix setzt in diesen kompartimentierten Bereich antibiotische Wirkstoffe frei, um die Infektion lokal in diesem Bereich zu behandeln.

In Ausführungsformen der erfindungsgemäßen Herzklappenprothese sind die Kompartimentierungsclips so angebracht, dass die Bögen (z.B. bei, Schneiden aus einem Materialstück) nicht auf den "Spitzen", sondern in den "Tälern" von rautenartigen Strukturen entspringen und nach oben weisen und dann durch die im Formgebungsprozess eingeprägte Form im expandierten Zustand fast kreisförmig und näherungsweise tangential nach außen weisen und dann an der Peripherie des Stentgerüstes nach unten laufend einen Bogen bilden. Dabei wird den Bögen eine 360°-Torsion eingeprägt, die ermöglicht, dass das Material der Bögen bei dem Prozess des nach oben Biegens vor der Implantation sowie während des nach unten Freisetzens bei der Implantation die auftretenden Verformungen und die damit einhergehenden Kräfte und Spannungen aufnimmt ohne zu brechen oder plastisch zu verformen.

In anderen Ausführungsformen der erfindungsgemäßen Herzklappenprothese reichen die Schenkel der Bögen der Kompartimentierungsclips nach dem Formgebungsprozess weit nach oben und sind dann über einen geeigneten Krümmungsradius nach außen oder näherungsweise tangential im 180° nach unten umgeklappt wobei das unterste Stück für die Implantation wieder nach oben geklappt wird, um diesen Bereich der Kompartimentierungsclips während der Implantation nicht auf gleicher axialer Höhe wie die innerhalb des Stentgerüsts angebrachten Herzklappensegel zu positionieren (Umfangsreduzierung während der Implantation).

Weiterhin können an Ausführungsbeispielen der erfindungsgemäßen Herzklappenprothese nach oben (axial) ausgerichtete Stabilisierungsbögen befestigt sein, welche bei der Implantation freigesetzt werden und in mindestens teilweise expandiertem Zustand die Herzklappenprothese in dem zu implantierenden Gefäß (zentral) positionieren, sodass die Bögen der Kompartimentierungsclips in alle nativen Herzklappensegel geführt werden können. In Ausführungsbeispielen der erfindungsgemäßen Herzklappenprothese können diese Zentralisierungsbögen im axial oberen Bereich durch Bogen-, Rauten- oder andersartige Elemente verbunden sein, um diesen Bereich der Herzklappenprothese mit einer größeren Radialkraft auszustatten. Zudem sind die erfindungsgemäßen Herzklappenprothesen zusammen mit dem Einführkathetersystem so ausgebildet, dass während der Implantation eine axiale Drehung der Herzklappenprothese möglich ist, um die Bögen der Kompartimentierungsclips in die nativen Herzklappensegel einzuführen.

### Kurze Beschreibung der Zeichnungen

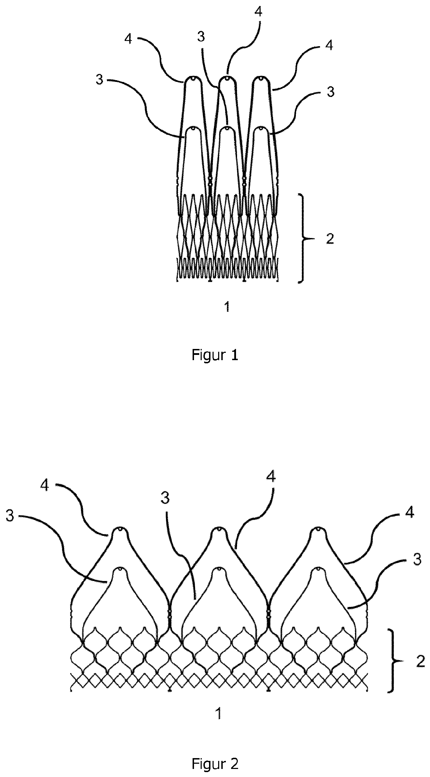

Figur 1 zeigt den geschnittenen Metallrahmen eines Ausführungsbeispiels mit dreizähliger Rotationssymmetrie der Herzklappenprothese zum Einsatz in einer dreisegeligen Herzklappe. Dargestellt ist das auf zwei Dimensionen gebrachte Schnittmuster für einen rohrförmigen Werkstoff.
Figur 2 zeigt den Rahmen der Herzklappenprothese des Ausführungsbeispiels aus Figur 1 in einer zweidimensionalen Darstellung, nachdem es im Laufe des Formgebungsprozesses radial aufgeweitet wurde und die Kompartimentierungsclips nach unten geklappt sind.

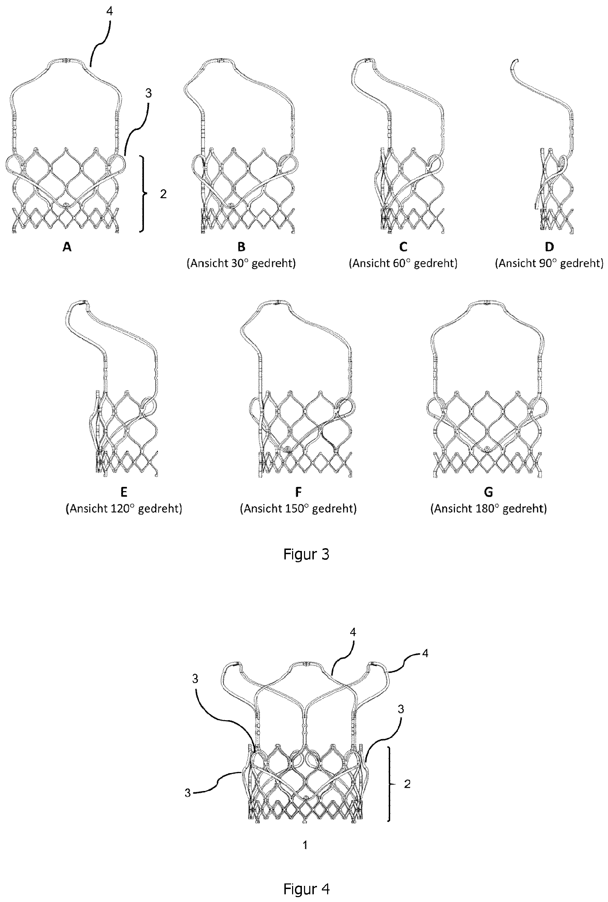

Figur 3 zeigt ein Drittel des Rahmens der Herzklappenprothese des Ausführungsbeispiels aus Figur 1, nach dem Formgebungsprozess, wobei die einzelnen Teile der Figur (A bis G) jeweils dasselbe Teil darstellen und die Ansicht in jeder Teilabbildung um 30° weiter in axialer Richtung gedreht ist.
Figur 4 zeigt eine seitliche Ansicht des Rahmens der Herzklappenprothese eines Ausführungsbeispiels aus Figur 1.

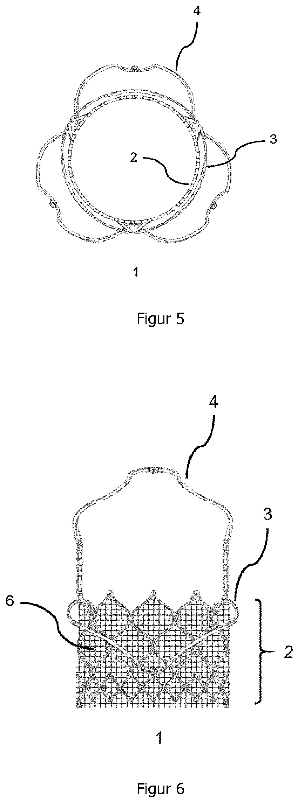

Figur 5 zeigt die Aufsicht auf den Rahmen der Herzklappenprothese eines Ausführungsbeispiels.
Figur 6 zeigt ein Drittel eines Ausführungsbeispiels, in dem der untere Teil des Stentgerüsts, nicht aber die Kompartimentierungsclips mit einer dünnen Schicht zur Abgrenzung gegen den Blutstrom und/oder die wirkstoffhaltigen Bereiche angebracht sind.

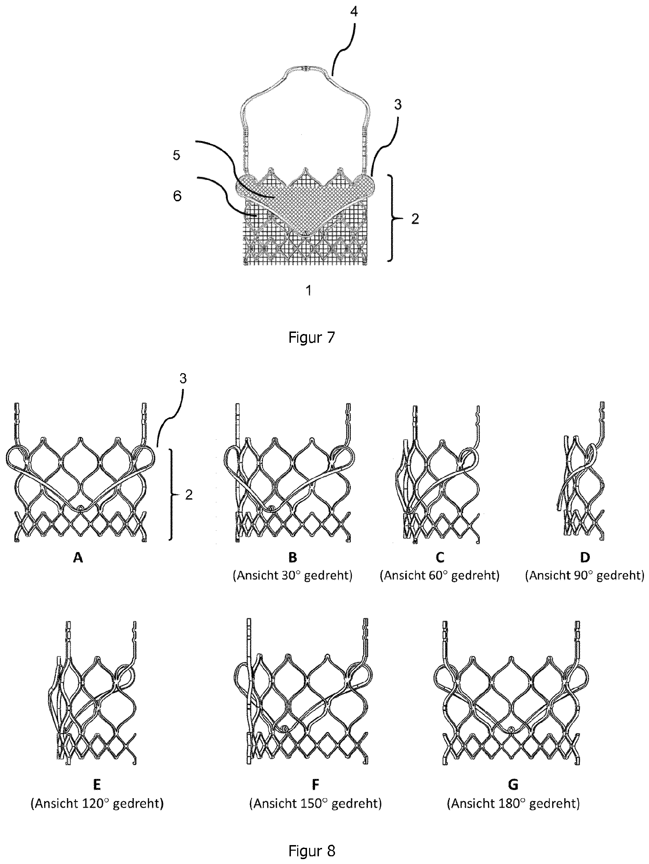

Figur 7 zeigt ein Drittel eines Ausführungsbeispiels, in dem sowohl der untere Teil des Stentgerüsts, als auch die Kompartimentierungsclips flächig mit einer dünnen Schicht zur Abgrenzung gegen den Blutstrom und/oder die wirkstoffhaltigen Bereiche angebracht sind.
Figur 8 zeigt ein Drittel eines weiteren Ausführungsbeispiels des Gerüsts, wobei die das Gerüst der Herzklappenprothese ohne Stabilisierungsbögen ausgebildet ist.

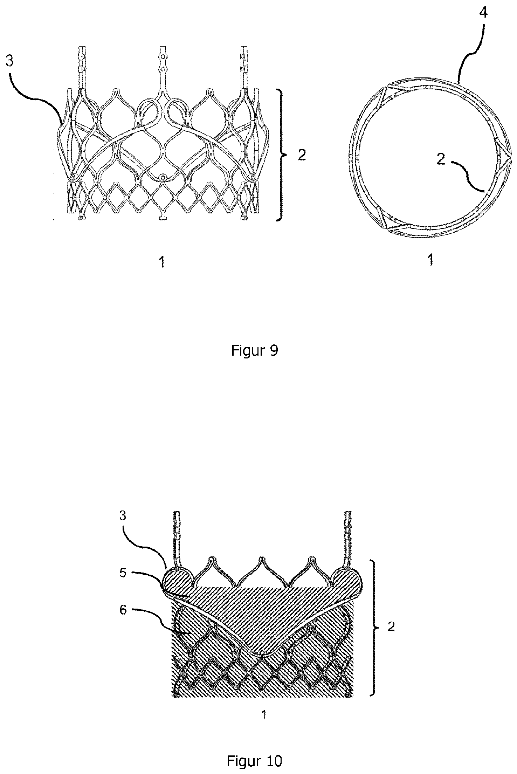

Figur 9 zeigt das gesamte Gerüst des Ausführungsbeispiels aus Figur 8, wobei eine Ansicht von vorne (frontal) und eine Aufsicht von oben (apikal) dargestellt ist.
Figur 10 zeigt einen Teil des Ausführungsbeispiels aus Figuren 8 und 9 mit der Trennschicht und den wirkstofffreisetzenden Matrices, die beide durchgehend am Stentgerüst beginnend bis über die Bögen der Kompartimentierungsclips reichen.

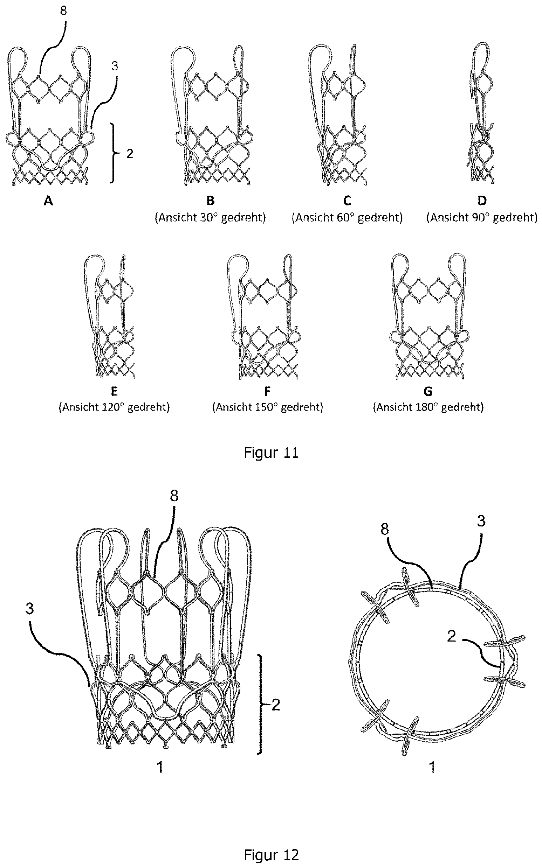

Figur 11 zeigt ein Drittel eines weiteren Ausführungsbeispiels des Gerüsts im expandierten und umgeklappten Zustand, wobei die Kompartimentierungsclips so ausgebildet sind, in einem langen Bogen vom Stentgerüst zuerst nach oben zu verlaufen und dann wieder nach unten zu reichen und wobei die vom Stentgerüst beginnende Trennschicht und wirkstofffreisetzenden Matrices nur im unteren Bereich der Kompartimentierungsclips angebracht sind. Weiterhin sind in diesem Ausführungsbeispiel die Bögen im oberen Bereich zur Stabilisierung und Aufbringung von Radialkraft durch Strukturen miteinander verbunden.
Figur 12 zeigt das gesamte Gerüst des Ausführungsbeispiels aus Figur 11, wobei eine Ansicht von vorne (frontal) und eine Aufsicht von oben (apikal) dargestellt ist.

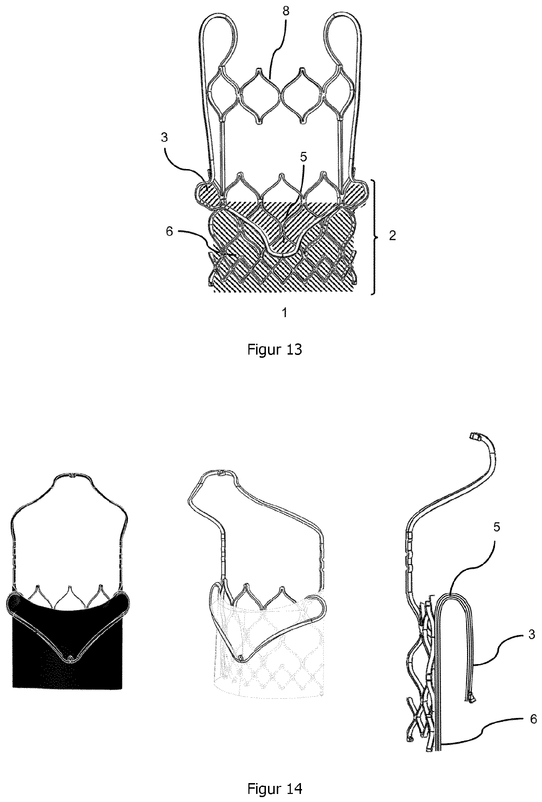

Figur 13 zeigt einen Teil des Ausführungsbeispiels aus Figuren 11 und 12 mit der Trennschicht und den wirkstofffreisetzenden Matrices, die beide durchgehend am Stentgerüst beginnend bis über die Bögen der Kompartimentierungsclips reichen.
Figur 14 zeigt in einem Schnittbild einen schematischen Aufbau der Lage von Stentgerüst mit den Bögen der Kompartimentierungsclips, einer daran befestigten Trennschicht und einer wirkstoffhaltigen Matrix.

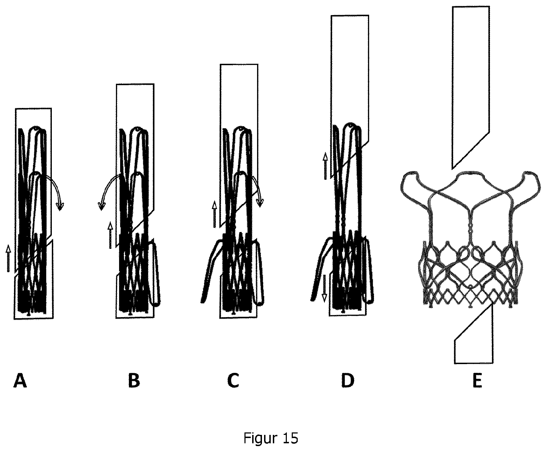

Figur 15 zeigt eine schematische Darstellung der Freisetzung eines Ausführungsbeispiels der erfindungsgemäßen Herzklappenprothese mittels eines an mindestens einem Teil angeschrägten Katheters, wodurch beim Zurückziehen der Katheterhülse bzw. Einführschleuse die Bögen der Kompartimentierungsclips nacheinander freigesetzt werden können.

### Bezugszeichenliste

- 1: Rahmen der Herzklappenprothese
- 2: Stentgerüst
- 3: Kompartimentierungsclips
- 4: Stabilisierungsbögen
- 5: Trennschicht
- 6: Wirkstoffhaltige Bereiche /Matrices

### Untenstehend sind weitere Varianten der vorliegenden Offenbarung gelistet.

1. Herzklappenprothese zur Beseitigung und Verhinderung bakterieller Besiedlungen an den Herzklappensegeln, insbesondere zum therapeutischen oder prophylaktischen Einsatz bei infektiöser Endokarditis, gekennzeichnet durch folgende Merkmale:
   - die Herzklappenprothese enthält ein aus Streben und Bögen aufgebautes radial komprimierbares Stentgerüst,
   - an dem Stentgerüst sind nach luminal (innen) mindestens zwei Herzklappensegel befestigt,
   - die Herzklappenprothese enthält mindestens zwei Kompartimentierungsclips, die im expandierten Zustand von distal (oben) nach außen (abluminal), d.h. entgegen der Blutflussrichtung bis nach proximal (unten) so geformt sind, dass die nativen Herzklappensegel und ggf. darauf befindliche Strukturen umgriffen werden, und das Stentgerüst in axialer Richtung zu positionieren und zu verankern,
   - die Herzklappenprothese weist mindestens eine Trennstruktur auf, welche mit dem Stentgerüst und den Kompartimentierungsclips verbunden ist und die im expandierten Zustand die zu behandelnden Bereiche mit den nativen Segeln vom Blutstrom trennt/kompartimentiert,
   - die Herzklappenprothese weist abluminal der mindestens einen Trennstruktur mindestens eine Matrix auf, welche antibiotische Wirkstoffe enthält und abgeben kann,
   - das Stentgerüst und/oder die Kompartimentierungsclips sind zusammen mit der mindestens einen Trennstruktur Struktur so ausgebildet, dass die Wirkstoffe aus den wirkstoffhaltigen Strukturen insbesondere in dem kompartimentierten Raum freigesetzt werden,
   - die Herzklappenprothese ist mittels einer geeigneten Vorrichtung, z.B. mittels eines Katheters, implantierbar.
2. Implantat nach Nummer 1, wobei das Stentgerüst und die Kompartimentierungsclips aus einem einzigen Stück eines komprimierbaren Werkstoffs gefertigt ist.
3. Implantat nach Nummer 2, wobei das Stentgerüst und die Kompartimentierungsclips aus einem selbstexpandierbaren Werkstoff bestehen.
4. Implantat nach Nummer 1, wobei das Stentgerüst und die Kompartimentierungsclips aus mindestens zwei miteinander verbundenen Stücken aus mindestens einem komprimierbaren Werkstoff gefertigt sind.
5. Implantat nach Nummer 4, wobei das Stentgerüst und die Kompartimentierungsclips aus mindestens zwei miteinander verbundenen Stücken eines selbstexpandierbaren Werkstoffs gefertigt sind.
6. Implantat nach Nummer 5, wobei das Stentgerüst und die Kompartimentierungsclips aus mindestens zwei miteinander verbundenen Stücken gefertigt sind, und das Stentgerüst aus eine ballonexpandierbaren Werkstoffs und die Kompartimentierungsclips aus einem selbstexpandierbaren Werkstoff gefertigt sind.
7. Implantat nach einer der Nummern 1 bis 6, wobei das Stentgerüst und/oder die Kompartimentierungsclips aus einer Formgedächtnislegierung bestehen.
8. Implantat nach einer der Nummern 1 bis 7, wobei die einzelnen Kompartimentierungsclips so ausgebildet sind, dass sie im Röntgenbild unterscheidbar sind.
9. Implantat nach einer der Nummern 1 bis 8, wobei die Anzahl der Kompartimentierungsclips ein ganzzahliges Vielfaches (n ≥ 1) von der Anzahl der Herzklappensegel ist, in welche die Klappe zu implantieren ist.
10. Implantat nach einer der Nummern 1 bis 9, wobei die Kompartimentierungsclips in einem teilweise expandierten Zustand so geformt und orientiert sind, nach abluminal-proximal (außen-unten) zu zeigen, sodass die einzelnen Bügel in den Raum zwischen Herzklappensegel und Gefäßwand geschoben werden können.
11. Implantat nach einer der Nummern 1 bis 10, wobei die Kompartimentierungsclips unterhalb der oberen Grenze der obersten Reihe von rautenartigen Strukturen ansetzen, damit die im expandierten Zustand bogenförmig nach unten reichenden Kompartimentierungsclips nicht oder nur wenig über den oberen Rand des Stentgerüstes reichen und dadurch den Blutzufluss und den Zugang zu den Eingängen von Koronargefäßen nicht versperren.
12. Implantat nach einer der Nummern 1 bis 11, wobei die Kompartimentierungsclips im komprimierten Zustand in Gänze oder teilweise im Einführkatheter so gegen die im Formgebungsprozess eingeprägte Richtung nach oben umgeklappt werden, dass sich die Bögen der Kompartimentierungsclips nicht auf der gleichen axialen Höhe mit dem Stentgerüst, sondern oberhalb des Stentgerüsts befinden.
13. Implantat nach Nummer 12, wobei die im komprimierten Zustand nach oben geklappten Kompartimentierungsclips bei der Freisetzung aus dem Einführkatheter selbständig (in Richtung der im Formgebungsprozess eingeprägten Form) nach unten umklappen, um auf der axialen Höhe des Stentgerüsts ausgerichtet zu sein.
14. Implantat nach einer der Nummern 1 bis 13, wobei die Kompartimentierungsclips im expandierten und nach unten umgeklappten Zustand so ausgebildet sind, dass die Segel der Herzklappe, in die die Herzklappenprothese zu implantieren ist, zwischen den Kompartimentierungsclips und dem Stentgerüst eingeklemmt werden, ohne den Kornarfluss zu beeinträchtigen.
15. Implantat nach einer der Nummern 1 bis 14, wobei die Kompartimentierungsclips im expandierten und umgeklappten Zustand so ausgebildet sind, dass sich jeweils die äußeren oberen Bereiche von jeweils zwei benachbarten Kompartimentierungsclips zugewandt sind, um möglichst große Bereiche der Segel einzuklemmen und zu kompartimentieren, insbesondere im Bereich der Kommissuren der zu ersetzenden Herzklappe.
16. Implantat nach einer der Nummern 1 bis 15, wobei die Kompartimentierungsclips so aus einem Rohr geschnitten werden, dass die Bögen vom Stentgerüst weg nach oben ausgerichtet sind und in einer Ebene von oben nach unten (um insgesamt 180°) gebogen wird.
17. Implantat nach einer der Nummern 1 bis 16, wobei die Kompartimentierungsclips so aus einem Rohr geschnitten werden, dass die Bögen vom Stentgerüst weg nach oben ausgerichtet sind und in einem Formgebungsprozess in der Weise nach unten gebogen werden, dass die Bögen im Bezug auf das Stentgerüst eine vollständige Drehung um 360° vollziehen. Anm.: Hierbei werden die Streben/Schenkel der Kompartimentierungsclips über die Schichtdicke (Schichtdicke entspricht der Materialstärke des Rohres) (nicht Strebenbreite) gebogen und um insgesamt 360° rotiert.
18. Implantat nach einer der Nummern 16 oder 17, wobei die Kompartimentierungsclips in alternativen Ausführungsformen so ausgebildet sind, dass sie weit nach distal (oben, stromabwärts) reichen, dass sie die Herzklappenprothese nach teilweiser Freisetzung zentral in dem zu implantierenden Blutgefäß ausrichtet und stabilisiert.
19. Implantat nach einer der Nummern 1 bis 18, wobei am Stentgerüst zusätzlich mindestens zwei selbstexpandierbare Bögen ausgebildet sind, welche beim Entlassen des oberen Teils der Herzklappenprothese freigesetzt werden und nach außen und oben gerichtet sind und die die gesamte Herzklappenprothese zentral in dem Blutgefäß ausrichten, durch das die Herzklappenprothese eingeführt wird.
20. Implantat nach Nummer 19, wobei die mindestens zwei Stabilisierungsbögen in ihrer Länge und/oder Form so ausgebildet sind, dass sie im Röntgenbild unterscheidbar sind und bei der Implantation nacheinander freigesetzt werden können.
21. Implantat nach Nummer 20, wobei der obere Teil der Kompartimentierungsclips durch Streben, Rauten oder andere Elemente verbunden ist, um im distalen Teil die radiale Stabilität zu vergrößern und eine Zentralisierung der Herzklappenprothese im zu implantierbaren Blutgefäß zu ermöglichen.
22. Implantat nach einer der Nummern 1 bis 19, wobei die mindestens eine wirkstoffhaltige Matrix bioresorbierbar ist.
23. Implantat nach einer der Nummern 1 bis 20, wobei die mindestens eine wirkstoffhaltige Matrix in Form von Folien ausgebildet ist.
24. Implantat nach einer der Nummern 1 bis 21, wobei mit den Kompartimentierungsclips zusammen mit den Trennschichten und der mindestens eine wirkstofffreisetzenden Matrix im teilweise expandierten Zustand regenschirmartig die an den nativen Klappensegeln anhaftenden infektiösen Vegetationen (zusammen mit den nativen Klappensegen selbst) bei der Implantation einfangen werden um diese zu kompartimentieren und um eine Embolisation der Vegetationen zu verhindern.
25. Implantat nach einer der Nummern 1 bis 24, wobei in einer Ausführung die wirkstoffhaltigen Matrix/Matrices an den Kompartimentierungsclips entfallen und diese Ausführungsform der postoperativen Infektionsprophylaxe dient.
26. Einführkatheter zum Einbringen der Herzklappenprothese nach einer der Nummern 1 bis 21, welcher im distalen Bereich der Einführschleuse eine Abschrägung oder Aussparung aufweist, wodurch die Kompartimentierungsclips der Herzklappenprothese beim Zurückziehen der Einführschleuse schrittweise nacheinander freigesetzt werden können.
27. Einführkatheter nach Nummer 22, wobei das Implantat nach einer der Nummern 1 bis 25 im Einführkatheter oder mit einem Teil des Katheters um die eigene Längsachse rotiert werden kann, um die Kompartimentierungsclips auf die Taschen der nativen Klappe auszurichten.
28. Herzklappenprothese zur Beseitigung und Verhinderung bakterieller Besiedlungen an den Herzklappensegeln, insbesondere zum therapeutischen oder prophylaktischen Einsatz bei infektiöser Endokarditis, gekennzeichnet durch folgende Merkmale:
   - die Herzklappenprothese enthält ein aus Streben, Bögen oder in sich geschlossenen Formen aufgebautes Stentgerüst,
   - an dem Stentgerüst sind nach luminal (innen) mindestens zwei Herzklappensegel befestigt,
   - die Herzklappenprothese enthält mindestens zwei Verankerungsclips, die von distal (oben) nach außen (abluminal), d.h. entgegen der Blutflussrichtung bis nach proximal (unten) geklappt werden können, um die nativen Herzklappensegel und ggf. darauf befindliche Strukturen zu (um)greifen,
   - die Herzklappenprothese weist abluminal Bereiche auf, die antibiotische Wirkstoffe enthalten und freisetzen können,
   - die Herzklappenprothese weist mindestens eine dünne Schicht auf, welche mit dem Stentgerüst und den Bügeln verbunden ist und die wirkstoffhaltigen Bereiche vom Blutstrom trennt
   - die Herzklappenprothese ist mittels eines Katheters implantierbar.
29. Implantat nach Nummer 1, wobei das Stentgerüst und die Verankerungsclips aus einem einzigen Stück eines selbstexpandierbaren Werkstoffs gefertigt sind.
30. Implantat nach einer der Nummern 28 oder 29, wobei das Stentgerüst selbstexpandierbar ist.
31. Implantat nach einer der Nummern 28 bis 30 wobei die Verankerungsclips aus einer Formgedächtnislegierung bestehen.
32. Implantat nach einer der Nummern 28 bis 31, wobei die einzelnen Verankerungsclips im Röntgenbild unterscheidbar sind.
33. Implantat nach einer der Nummern 28 bis 32, wobei die Anzahl der Verankerungsclips ein ganzzahliges Vielfaches (n ≥ 1) von der Anzahl der Herzklappensegel ist, in welche die Klappe zu implantieren ist.
34. Implantat nach einer der Nummern 28 bis 33, wobei die Verankerungsclips in einem teilweise expandierten Zustand so geformt und orientiert sind, nach abluminal-proximal (außen-unten) zu zeigen, so dass die einzelnen Bügel in den Raum zwischen Herzklappensegel und Gefäßwand geschoben werden können.
35. Implantat nach einer der Nummern 28 bis 34, wobei die Verankerungsclips unterhalb der oberen Grenze der obersten Reihe von rautenartigen Strukturen ansetzen, damit die im expandierten Zustand bogenförmig nach unten reichenden Verankerungsclips nicht oder nur wenig über den oberen Rand der obersten Reihe rautenartiger Strukturen reichen und dadurch den Blutzufluss und den Zugang zu den Eingängen von Koronargefäßen nicht versperren.
36. Implantat nach einer der Nummern 28 bis 35, wobei die Verankerungsclips im expandierten und umgeklappten Zustand so ausgebildet sind, die Segel der Herzklappe, in die die Herzklappenprothese zu implantieren ist, zwischen den Verankerungsclips und dem Stentgerüst einzuklemmen.
37. Implantat nach einer der Nummern 28 bis 36, wobei die Verankerungsclips im expandierten und umgeklappten Zustand so ausgebildet sind, dass sich jeweils die äußeren oberen Bereiche von jeweils zwei benachbarten Verankerungsclips zugewandt sind, um möglichst große Bereiche der Segel einzuklemmen und zu kompartimentieren, insbesondere im Bereich der Kommissuren der zu ersetzenden Herzklappe.
38. Implantat nach einer der Nummern 28 bis 37, wobei die mindesten eine dünne Schicht im expandierten Zustand zusammen mit den Verankerungsclips die Segel der Herzklappe, in die die Herzklappenprothese zu implantieren ist, umfasst und diese so vom Blutstrom abgrenzen oder kompartimentiert.
39. Implantat nach einer der Nummern 28 bis 38, wobei die Bereiche mit antibiotischen Wirkstoffen im expandierten Zustand zusammen mit den dünnen Schichten und den Verankerungsclips die Segel der Herzklappe, in die die Herzklappenprothese zu implantieren ist, umfassen und diese so vom Blutstrom abgrenzen oder kompartimentieren.
40. Implantat nach einer der Nummern 28 bis 39, wobei am Stentgerüst mindestens zwei expandierbare Stabilisierungsbögen ausgebildet sind, welche das die Herzklappenprothese beim Einbringen mittels Katheter zentral in dem Blutgefäß ausrichtet, durch das die Herzklappenprothese eingeführt wird.
41. Implantat nach einer der Nummern 28 bis 40, wobei das im weitesten Sinne zylindrische Stentgerüst der Herzklappenprothese eine so geringe Höhe aufweist, dass sie unterhalb des Abgangs der Koronararterien verbleibt und damit einen eventuell später notwendigen Zugang in die Koronararterien mittels eines Herzkatheters erleichtert.
42. Implantat nach einer der Nummern 28 bis 41, wobei die wirkstoffhaltigen Bereiche in Form von Folien ausgebildet sind.
43. Implantat nach einer der Nummern 28 bis 42, wobei die wirkstoffhaltigen Bereiche bioresorbierbar sind.
44. Implantat nach einer der Nummern 28 bis 43, wobei die Verankerungsclips zusammen mit den dünnen Schichten und wirkstofffreisetzenden Bereichen im teilweise expandierten Zustand regenschirmartig an den Klappen anhaftende infektiöse Vegetationen bei der Implantation einfangen und eine Embolisation verhindern.
45. Implantat nach einer der Nummern 28 bis 44, wobei bei der Implantation durch die nach unten zur Außenseite des Stentgerüstes ausgerichteten Verankerungsclips ein vom Blutstrom weitgehend abgeschirmtes Kompartiment entsteht, indem die antibiotischen Wirkstoffe zur Behandlung einer Endokarditis in hoher Konzentration freigesetzt werden und verbleiben können.
46. Implantat nach einer der Nummern 28 bis 45, wobei die mindestens 2 Stabilisierungsbögen (Nummer 19) in einer Ausführung auch weggelassen werden können.
47. Implantat nach einer der Nummern bis 28 bis 46, wobei in einer 3. Ausführung die wirkstoffhaltigen Bereiche an den Verankerungsclips entfallen und diese Ausführungsform der postoperativen Infektionsprophylaxe dient.
48. Einführkatheter zum Einbringen der Herzklappenprothese, welcher im distalen Bereich der Einführschleuse eine Abschrägung oder Aussparung aufweist, wodurch die Verankerungsclips der nichtexpandierten Herzklappenprothese schrittweise nacheinander freigesetzt werden können.
49. Einführkatheter nach Nummer 48, wobei das Implantat nach einer der Nummern 1 bis 25 mittels im oder mit einem Teil des Katheters um die eigene Längsachse rotiert werden kann, um die Verankerungsclips auf die Taschen der nativen Klappe auszurichten.
50. Verankerung für Herzklappenprothesen zur Behandlung und/oder zum Ersatz einer an einer Entzündung und/oder Infektion erkrankten Herzklappe, wobei die Verankerung, in einem komprimierten Zustand, minimal-invasiv in den Körper des Patienten einführbar und im Implantationsort an der erkrankten Herzklappe in einen expandierten Zustand überführbar ist und folgendes aufweist:
   - ein Stentgerüst (2) welches dazu ausgebildet ist mindestens zwei Herzklappensegel an einer Innenseite zu befestigen, wobei das Stentgerüst ein proximales Ende und ein distales Ende aufweist;
   - mindestens zwei Kompartimentierungsclips (3), welche mit dem Stentgerüst verbunden sind und dazu ausgebildet sind, im komprimierten Zustand der Verankerung, über das distale Ende des Stentgerüsts (2) hinauszuragen, sowie, im expandierten Zustand der Herzklappenprothese, das Stentgerüst (2) zumindest teilweise überlappen,
   wobei die Kompartimentierungsclips (3) jeweils mindestens einen Arm aufweisen, welcher dazu ausgebildet ist sich bei einem Übergang zwischen dem komprimierten Zustand und dem expandierten Zustand umzustülpen und zu verdrillen.
51. Verankerung nach Nummer 50, wobei die Kompartimentierungsclips (3) ein proximales Ende, das mit dem distalen Ende des Stentgerüsts (2) verbunden ist, und ein distales Ende, das im komprimierten Zustand über distale Ende des Stentgerüsts hinausragt, aufweisen, wobei die Kompartimentierungsclips (2) eine erste Oberfläche und eine der ersten Oberfläche gegenüber liegende zweite Oberfläche aufweisen, und wobei die erste Oberfläche am distalen Ende der Kompartimentierungsclips, im komprimierten Zustand und im expandierten Zustand, im Wesentlichen radial nach innen gerichtet ist.
52. Verankerung nach Nummer 51, wobei die zweite Oberfläche am distalen Ende der Kompartimentierungsclips, im komprimierten Zustand und im expandierten Zustand, im Wesentlichen radial nach außen gerichtet ist.
53. Verankerung nach einem der Nummern 50 bis 52, wobei die Verankerung dazu ausgebildet ist, im expandierten Zustand, native Segel der erkrankten Herzklappe, zwischen dem Stentgerüst und den Kompartimentierungsclips (3) zu halten, insbesondere einzuklemmen.
54. Verankerung nach einem der Nummern 50 bis 53, welche eine Trennstruktur aufweist, die derart mit dem Stentgerüst (2) und den Kompartimentierungsclips (3) verbunden ist, dass die Trennstruktur, im expandierten Zustand, die zu behandelnden Bereiche der nativen Segel vom Blutstrom trennt.
55. Verankerung nach Nummer 54, wobei die Trennstruktur zumindest außenseitig eine Matrix aufweist, welche antibiotische Wirkstoffe enthält und abgeben kann.
56. Verankerung nach einem der Nummern 50 bis 55, wobei das Stentgerüst und die Kompartimentierungsclips aus einem Stück gefertigt sind.
57. Verankerung nach einem der Nummern 50 bis 56, wobei das Stentgerüst und/oder die Kompartimentierungsclips aus einem komprimierbaren Werkstoff gefertigt sind.
58. Verankerung nach einem der Nummern 50 bis 57, wobei das Stentgerüst und/oder die Kompartimentierungsclips aus einem selbstexpandierenden Werkstoff gefertigt sind.
59. Verankerung nach einem der Nummern 50 bis 55, wobei das Stentgerüst und die Kompartimentierungsclips aus mindestens zwei miteinander verbundenen oder verbindbaren Stücken eines selbstexpandierbaren Werkstoffs gefertigt sind.
60. Verankerung nach einem der Nummern 50 bis 55, wobei das Stentgerüst und die Kompartimentierungsclips aus mindestens zwei miteinander verbundenen verbindbaren Stücken gefertigt sind, und das Stentgerüst aus einem ballonexpandierbaren Werkstoff und die Kompartimentierungsclips aus einem selbstexpandierbaren Werkstoff gefertigt sind.
61. Verankerung nach einem der Nummern 50 bis 60, wobei das Stentgerüst und/oder die Kompartimentierungsclips aus einer Formgedächtnislegierung, insbesondere Nitinol, bestehen.
62. Verankerung nach einem der Nummern 50 bis 61, wobei die mindestens zwei Kompartimentierungsclips (3), im expandierten Zustand der Verankerung, das Stentgerüst (2) derart überlappen, dass kein Teil der Kompartimentierungsclips über das distale Ende des Stentgerüsts (2) hinausragt.
63. Katheter zum Einführen einer Verankerung für Herzklappenprothesen in den Körper eines Patienten, wobei die Verankerung in einem komprimierten Zustand, mit Hilfe des Katheters, minimal-invasiv in den Körper des Patienten einführbar und im Implantationsort an der erkrankten Herzklappe in einen expandierten Zustand überführbar ist und die Verankerung folgendes aufweist:
   - ein Stentgerüst (2) welches dazu ausgebildet ist mindestens zwei Herzklappensegel an einer Innenseite zu befestigen, wobei das Stentgerüst ein proximales Ende und ein distales Ende aufweist;
   - mindestens zwei Kompartimentierungsclips (3), welche mit dem Stentgerüst verbunden sind und, im komprimierten Zustand der Verankerung, über das distale Ende des Stentgerüsts (2) hinausragen, sowie, im expandierten Zustand der Verankerung, das Stentgerüst (2) zumindest teilweise überlappen,
   wobei der Katheter eine Katheterspitze aufweist, welche über eine Handhabe des Katheters (20) derart manipulierbar ist, dass das Implantat (1) schrittweise von der Katheterspitze freisetzbar ist.
64. Katheter nach Nummer 63, wobei die Katheterspitze derart ausgebildet ist, dass die mindestens zwei Kompartimentierungsclips (3) schrittweise, nacheinander von der Katheterspitze freisetzbar sind.
65. Katheter nach Nummer 64, wobei die Katheterspitze eine angeschrägte Hülse aufweist, welche dazu ausgebildet ist die Verankerung während dem Einbringen vollständig zu überdecken und mit Hilfe der Handhabe zurückziehbar ist, um die Kompartimentierungsclips schrittweise freizusetzen.
66. Katheter nach einem der Nummern 63 bis 66, wobei der Katheter dazu ausgebildet ist die Verankerung, über die Handhabe, zu rotieren, um Kompartimentierungsclips auf die Taschen/Segel der nativen Klappe auszurichten.
67. System zur Behandlung oder zum Ersatz einer an einer entzündeten, thrombosierten oder degenerierten Herzklappe mit einer Verankerung nach einem der Nummern 50 bis 62 und einem Katheter nach einem der Nummern 63 bis 66, wobei die Verankerung dazu ausgebildet ist, insbesondere in ihrem komprimierten Zustand, in der Katheterspitze aufnehmbar zu sein, und wobei die Katheterspitze ausgebildet ist, die Verankerung (1), insbesondere in ihrem komprimierten Zustand, aufzunehmen.
68. Verfahren zur Behandlung oder zum Ersatz einer an einer entzündeten, thrombosierten oder degenerierten Herzklappe, wobei das Verfahren folgendes aufweist:
   - Einführen einer Verankerung nach einem der Nummern 50 bis 62 mit Hilfe eines Katheters nach einem der Nummern 63 bis 66;
   - Freisetzen der Kompartimentierungsclips (3);
   - Ausrichten der Kompartimentierungsclips auf einer ersten Seite der nativen Segel der erkrankten Herzklappe;
   - Freisetzen des Stentgerüsts (2) auf einer zweiten Seite der nativen Segel, welche der ersten Seite gegenüberliegt.
69. Verfahren nach Nummer 68, wobei das Verfahren ferner den Verfahrensschritt der Freigabe von antimikrobiellen, antithrombotischen und zellwachstumshemmenden Wirkstoffen aufweist, wobei die Freigabe in-situ erfolgt.
70. Verfahren nach Nummer 68 oder 69, wobei die Kompartimentierungsclips (3) schrittweise, nacheinander freigesetzt werden.
71. Verankerung für Herzklappenprothesen zur Behandlung und/oder zum Ersatz einer an einer Entzündung und/oder Infektion erkrankten Herzklappe, wobei die Verankerung in einem komprimierten Zustand minimal-invasiv in den Körper des Patienten einführbar und im Implantationsort an der erkrankten Herzklappe in einen expandierten Zustand überführbar ist und folgendes aufweist:
   - ein Stentgerüst (2) welches dazu ausgebildet ist mindestens zwei Herzklappensegel an einer Innenseite zu befestigen, wobei das Stentgerüst ein proximales Ende und ein distales Ende aufweist;
   - mindestens zwei Stabilisierungsbögen (4) welche über das distale Ende des Stentgerüsts (2) hinausragen und dazu ausgebildet sind sich, im expandierten Zustand der Verankerung, derart an einer Gefäßwand abzustützen, dass die Verankerung, nach Freisetzung der Stabilisierungsbögen (4), zentral in dem zu implantierenden Blutgefäß ausgerichtet ist.
72. Verankerung nach Nummer 71, wobei die mindestens zwei Stabilisierungsbögen in ihrer Länge und/oder Form so ausgebildet sind, dass sie im Röntgenbild unterscheidbar sind und bei der Implantation nacheinander freigesetzt werden können.
73. Verankerung nach Nummer 71 oder 72, wobei die Stabilisierungsbögen ein proximales Ende, das mit dem distalen Ende des Stentgerüsts (2) verbunden ist, und ein distales Ende, das über distale Ende des Stentgerüsts hinausragt, aufweisen, und wobei die Stabilisierungsbögen am distalen Ende durch Streben, Rauten oder andere Elemente miteinander verbunden sind, welche dazu ausgebildet sind die radiale Stabilität zu vergrößern und eine Zentralisierung der Verankerung im zu implantierbaren Blutgefäß zu ermöglichen.
74. Verankerung nach einem der Nummern 71 bis 73, welche eine Trennstruktur aufweist, die derart mit dem Stentgerüst (2) verbunden ist, dass die Trennstruktur, im expandierten Zustand, die zu behandelnden Bereiche der nativen Segel vom Blutstrom trennt.
75. Verankerung nach Nummer 74, wobei die Trennstruktur zumindest außenseitig eine Matrix auf aufweist, welche antibiotische Wirkstoffe enthält und abgeben kann.
76. Verankerung nach einem der Nummern 71 bis 75, wobei das Stentgerüst und die Stabilisierungsbögen aus einem Stück gefertigt sind.
77. Verankerung nach einem der Nummern 71 bis 76, wobei das Stentgerüst und/oder die Stabilisierungsbögen aus einem komprimierbaren Werkstoff gefertigt sind.
78. Verankerung nach einem der Nummern 71 bis 77, wobei das Stentgerüst und/oder die Stabilisierungsbögen aus einem selbstexpandierenden Werkstoff gefertigt sind.
79. Verankerung nach einem der Nummern 71 bis 78, wobei das Stentgerüst und/oder die Stabilisierungsbögen aus einer Formgedächtnislegierung, insbesondere Nitinol, bestehen.
80. Verankerung für Herzklappenprothesen zur Behandlung und/oder zum Ersatz einer an einer Entzündung und/oder Infektion erkrankten Herzklappe, wobei die Verankerung, in einem komprimierten Zustand, minimal-invasiv in den Körper des Patienten einführbar und im Implantationsort an der erkrankten Herzklappe in einen expandierten Zustand überführbar ist und folgendes aufweist:
   - ein Stentgerüst (2) welches dazu ausgebildet ist mindestens zwei Herzklappensegel an einer Innenseite zu befestigen, wobei das Stentgerüst ein proximales Ende und ein distales Ende aufweist;
   - mindestens zwei Kompartimentierungsclips (3), welche mit dem Stentgerüst verbunden sind und dazu ausgebildet sind, im komprimierten Zustand der Verankerung, über das distale Ende des Stentgerüsts (2) hinauszuragen, sowie, im expandierten Zustand der Verankerung, das Stentgerüst (2) zumindest teilweise überlappen,
   - eine Trennstruktur, die derart mit dem Stentgerüst (2) und den Kompartimentierungsclips (3) verbunden ist, dass die Trennstruktur, im expandierten Zustand, die zu behandelnden Bereiche der nativen Segel vom Blutstrom trennt.
81. Verankerung nach Nummer 80, wobei die Trennstruktur zumindest außenseitig eine Matrix aufweist, welche antibiotische Wirkstoffe enthält und abgeben kann.
82. Verankerung nach Nummer 81, wobei die mindestens eine wirkstoffhaltige Matrix bioresorbierbar ist.
83. Verankerung nach einem der Nummern 80 bis 82, wobei die Verankerung dazu ausgebildet ist, im expandierten Zustand, native Segel der erkrankten Herzklappe, zwischen dem Stentgerüst und den Kompartimentierungsclips (3) zu halten, insbesondere einzuklemmen.
84. Verankerung nach einem der Nummern 80 bis 83, wobei das Stentgerüst und die Kompartimentierungsclips aus einem Stück gefertigt sind.
85. Verankerung nach einem der Nummern 80 bis 84, wobei das Stentgerüst und/oder die Kompartimentierungsclips aus einem komprimierbaren Werkstoff gefertigt sind.
86. Verankerung nach einem der Nummern 80 bis 85, wobei das Stentgerüst und/oder die Kompartimentierungsclips aus einem selbstexpandierenden Werkstoff gefertigt sind.
87. Verankerung nach einem der Nummern 80 bis 86, wobei das Stentgerüst und die Kompartimentierungsclips aus mindestens zwei miteinander verbundenen oder verbindbaren Stücken eines selbstexpandierbaren Werkstoffs gefertigt sind.
88. Verankerung nach einem der Nummern 80 bis 87, wobei das Stentgerüst und die Kompartimentierungsclips aus mindestens zwei miteinander verbundenen verbindbaren Stücken gefertigt sind, und das Stentgerüst aus einem ballonexpandierbaren Werkstoff und die Kompartimentierungsclips aus einem selbstexpandierbaren Werkstoff gefertigt sind.
89. Verankerung nach einem der Nummern 80 bis 88, wobei das Stentgerüst und/oder die Kompartimentierungsclips aus einer Formgedächtnislegierung, insbesondere Nitinol, bestehen.
90. Verankerung nach einem der Nummern 80 bis 89, wobei die Kompartimentierungsclips (3) jeweils mindestens einen Arm aufweisen, welcher dazu ausgebildet ist sich bei einem Übergang zwischen dem komprimierten Zustand und dem expandierten Zustand umzustülpen und zu verdrillen.
91. Verankerung nach Nummer 90, wobei die mindestens zwei Kompartimentierungsclips (3), im expandierten Zustand der Verankerung, das Stentgerüst (2) derart überlappen, dass kein Teil der Kompartimentierungsclips über das distale Ende des Stentgerüsts (2) hinausragt.
92. Verankerung nach einem der Nummern 50 bis 62 oder 71 bis 91, wobei die Verankerung drei Kompartimentierungsclips (3) aufweist.
93. Verankerung nach einem der Nummern 50 bis 62 oder 71 bis 92, wobei die mindestens zwei Kompartimentierungsclips (3) im expandierten Zustand radial voneinander beabstandet und über das Stentgerüst (2) miteinander verbunden sind.
94. Verankerung nach einem der Nummern 50 bis 62 oder 71 bis 93, wobei das Stentgerüst an seinem proximalen Ende eine erste Zellstruktur aus Streben und an seinem distalen Ende eine zweite Zellstruktur aus Streben aufweist, wobei die erste und die zweite Zellstruktur unterschiedlich ausgebildet sind.
95. Verankerung nach Nummer 94, wobei die erste Zellstruktur eine höhere Dichte an Streben aufweist als die zweite Zellstruktur.
96. Verankerung nach einem der Nummern 50 bis 62 oder 71 bis 95, wobei die Verankerung aus einem Rohr aus Formgedächtnislegierung geschnitten ist.

## Patentansprüche

1. Verankerung für Herzklappenprothesen zum Ersatz einer Herzklappe, insbesondere einer degenerierten Herzklappe, wobei die Verankerung eine Grundform aufweist und zum minimal-invasiven Einführen der Verankerung in den Körper des Patienten reversibel und durch elastische Umformung und/oder Kompression zumindest von Bereichen oder Komponenten der Verankerung in eine Zuführungsform überführbar ist, wobei die Verankerung folgendes aufweist:
• ein Stentgerüst (2), welches dazu ausgebildet ist, mindestens zwei Herzklappensegel an einer Innenseite zu befestigen, wobei das Stentgerüst ein proximales Ende und ein distales Ende aufweist,
**dadurch gekennzeichnet, dass**
die die Verankerung ferner mindestens zwei Kompartimentierungsclips (3) aufweist, welche jeweils an einem ersten Endbereich mit dem Stentgerüst verbunden sind und einen freien, zweiten Endbereich aufweisen, wobei in der Grundform der Verankerung die Kompartimentierungsclips (3) eine Bogenform zwischen den ersten und zweiten Endbereichen aufweisen.

2. Verankerung nach Anspruch 1,
wobei in der Grundform der Verankerung die Kompartimentierungsclips (3) das Stentgerüst zumindest teilweise überlappen, insbesondere in radialer und proximaler (herznaher) Richtung des Stentgerüsts; und/oder
wobei in der Grundform der Verankerung die mindestens zwei Kompartimentierungsclips (3) das Stentgerüst (2) derart überlappen, dass kein Teil der Kompartimentierungsclips über das distale Ende des Stentgerüsts (2) hinausragt.

3. Verankerung nach Anspruch 1 oder 2,
wobei die Kompartimentierungsclips (3) derart elastisch umklappbar sind, dass sich in der Zuführungsform der Verankerung die Kompartimentierungsclips zumindest im Wesentlichen flach zwischen den ersten und zweiten Endbereichen erstrecken.

4. Verankerung nach einem der Ansprüche 1 bis 3,
wobei die Kompartimentierungsclips Arme aufweisen, welche in der Grundform der Verankerung zumindest bereichsweise verdrillt sind, wobei die Kompartimentierungsclips (2) vorzugsweise eine erste Clipoberfläche und eine der ersten Clipoberfläche gegenüberliegende zweite Clipoberfläche aufweisen, und wobei sowohl in der Grundform als auch in der Zuführungsform der Verankerung die erste Clipoberfläche am freien, zweiten Endbereich der Kompartimentierungsclips zumindest im Wesentlichen radial nach innen, insbesondere in Richtung einer Längsachse der Verankerung, gerichtet ist; oder
wobei die Kompartimentierungsclips (2) eine erste Clipoberfläche und eine der ersten Clipoberfläche gegenüberliegende zweite Clipoberfläche aufweisen, und wobei in der Grundform die erste Clipoberfläche nach außen und die zweite Clipoberfläche nach innen und in der Zuführungsform die erste Oberfläche nach innen und die zweite Oberfläche nach außen gerichtet ist.

5. Verankerung nach einem der Ansprüche 1 bis 4,
wobei die Verankerung dazu ausgebildet ist, in einem implantierten Zustand der Verankerung native Segel bzw. Leaflets der nativen Herzklappe zwischen dem Stentgerüst und den Kompartimentierungsclips (3) zu halten, insbesondere einzuklemmen.

6. Verankerung nach einem der Ansprüche 1 bis 5,
wobei das Stentgerüst und die Kompartimentierungsclips aus einem Stück gefertigt sind; und/oder
wobei das Stentgerüst und/oder die Kompartimentierungsclips aus einem komprimierbaren Werkstoff gefertigt sind; und/oder
wobei das Stentgerüst und/oder die Kompartimentierungsclips aus einem selbstexpandierenden Werkstoff gefertigt sind; und/oder
wobei das Stentgerüst und/oder die Kompartimentierungsclips aus einer Formgedächtnislegierung, insbesondere Nitinol, bestehen; und/oder
wobei die Verankerung aus einem Rohr aus Formgedächtnislegierung geschnitten ist.

7. Verankerung nach einem der Ansprüche 1 bis 5,
wobei das Stentgerüst und die Kompartimentierungsclips aus mindestens zwei miteinander verbundenen oder verbindbaren Stücken eines selbstexpandierbaren Werkstoffs gefertigt sind, und wobei vorzugsweise das Stentgerüst aus einem ballonexpandierbaren Werkstoff und die Kompartimentierungsclips aus einem selbstexpandierbaren Werkstoff gefertigt sind.

8. Verankerung nach einem der Ansprüche 1 bis 7 oder nach dem Oberbegriff von Anspruch 1,
**dadurch gekennzeichnet, dass**
die Verankerung mindestens zwei Stabilisierungsbögen (4) aufweist, welche über das distale Ende des Stentgerüsts (2) hinausragen und dazu ausgebildet sind, sich im implantierten Zustand der Verankerung derart an einer Gefäßwand abzustützen, dass die Verankerung nach Freisetzung der Stabilisierungsbögen (4) zentral in dem zu implantierenden Blutgefäß ausgerichtet ist.

9. Verankerung nach Anspruch 8,
wobei die mindestens zwei Stabilisierungsbögen in ihrer Länge und/oder Form so ausgebildet sind, dass sie im Röntgenbild unterscheidbar sind und bei der Implantation nacheinander freigesetzt werden können.

10. Verankerung nach Anspruch 8 oder 9,
wobei die Stabilisierungsbögen ein proximales Ende, das mit dem distalen Ende des Stentgerüsts (2) verbunden ist, und ein distales Ende, das über distale Ende des Stentgerüsts hinausragt, aufweisen, und wobei die Stabilisierungsbögen am distalen Ende durch Streben, Rauten oder andere Elemente miteinander verbunden sind, welche dazu ausgebildet sind, die radiale Stabilität zu vergrößern und eine Zentralisierung der Verankerung im zu implantierbaren Blutgefäß zu ermöglichen (8).

11. Verankerung nach einem der Ansprüche 8 bis 10,
wobei das Stentgerüst und die Stabilisierungsbögen aus einem Stück gefertigt sind; und/oder
wobei das Stentgerüst und/oder die Stabilisierungsbögen aus einem komprimierbaren Werkstoff gefertigt sind; und/oder
wobei das Stentgerüst und/oder die Stabilisierungsbögen aus einem selbstexpandierenden Werkstoff gefertigt sind; und/oder
wobei das Stentgerüst und/oder die Stabilisierungsbögen aus einer Formgedächtnislegierung, insbesondere Nitinol, bestehen; und/oder
wobei die Verankerung aus einem Rohr aus Formgedächtnislegierung geschnitten ist.

12. Verankerung nach einem der Ansprüche 1 bis 11,
wobei das Stentgerüst an seinem proximalen Ende eine erste Zellstruktur aus Streben und an seinem distalen Ende eine zweite Zellstruktur aus Streben aufweist, wobei die erste und die zweite Zellstruktur unterschiedlich ausgebildet sind, und wobei die erste Zellstruktur vorzugsweise eine höhere Dichte an Streben aufweist als die zweite Zellstruktur.

13. Herzklappenprothese, insbesondere zum Ersatz einer Herzklappe, insbesondere einer degenerierten Herzklappe, wobei die Herzklappenprothese folgendes aufweist:
• eine Verankerung nach einem der Ansprüche 1 bis 12; und
• mindestens zwei Herzklappensegel, welche an der Innenseite des Stentgerüsts der Verankerung befestigt sind.

14. Katheter zum Einführen einer Verankerung für Herzklappenprothesen in den Körper eines Patienten, wobei mit Hilfe des Katheters die Verankerung in einer Zuführungsform minimal-invasiv in den Körper des Patienten einführbar und im Implantationsort an der erkrankten Herzklappe in einen explantierten Zustand überführbar ist, und wobei die Verankerung eine Verankerung nach einem der Ansprüche 1 bis 12 ist, und wobei der Katheter eine Katheterspitze aufweist, welche über eine Handhabe des Katheters (20) derart manipulierbar ist, dass das Implantat (1) schrittweise von der Katheterspitze freisetzbar ist.

15. Katheter nach Anspruch 14,
wobei die Katheterspitze derart geteilt ausgebildet ist, dass die mindestens zwei Kompartimentierungsclips (3) schrittweise, nacheinander von der Katheterspitze freisetzbar sind, und dann folgend das Stentgerüst freigesetzt werden kann;
wobei die geteilte Katheterspitze vorzugsweise eine angeschrägte Hülse aufweist, welche dazu ausgebildet ist, die Verankerung während des Einbringens zu überdecken, und welche mit Hilfe der Handhabe zurückziehbar ist, um die Kompartimentierungsclips schrittweise freizusetzen; und/oder
wobei der Katheter vorzugsweise dazu ausgebildet ist, die Verankerung über die Handhabe zu rotieren, um Kompartimentierungsclips auf die Taschen/Segel/Leaflets der nativen Klappe auszurichten.
